# EUROPEAN PATENT APPLICATION

(11) **EP 1 700 641 A1**
(43) Date of publication of application: **13.09.2006**
(21) Application number: 06004728.9
(22) Date of filing: 08.03.2006
(51) Int. Cl.: B06B 1/06

(54) **Endocavity ultrasonic probe**

(30) Priority: 09.03.2005 JP 2005065392
(71) Applicant: FUJI PHOTO FILM CO., LTD., Minami-Ashigara-shi, Kanagawa-ken (JP)
(72) Inventor: Wada, Mitsugu, Kaisei-machi, Ashigarakami-gun Kanagawa (JP)
(74) Representative: Klunker . Schmitt-Nilson . Hirsch

(57) **Abstract**

An endocavity ultrasonic probe (2) includes a backing member (4) of cylindrical or convex shape, and a plurality of strip shaped ultrasonic transducers (5) arranged on the backing member. The ultrasonic transducer is composed of three piezoelectric layers (10), electrode layers (11a, 11b) formed above and below each piezoelectric layer, insulating films (12) formed at one side face of each electrode layer, and side electrodes (13a, 13b) formed along both side faces of the piezoelectric layers and the electrode layers. The electrode layers are electrically connected to one of the side electrodes but insulated from the other side electrode. Two vertically adjacent electrode layers are electrically connected to a different one of the side electrodes.

## Description

### BACKGROUND OF THE INVENTION

### FIELD OF THE INVENTION

The present invention relates to an endocavity ultrasonic probe, which is inserted into a body cavity for radiating ultrasonic waves to an internal body part and receiving the echoes thereof.

### BACKGROUND ARTS

Recently, in the field of medicine, ultrasonic images are more introduced for diagnosis. The ultrasonic image is obtained by radiating ultrasonic waves from an ultrasonic probe toward a body part of a patient and electrically detecting the echo (reflection) from the body part. Most of the ultrasonic proves are provided with a plurality of ultrasonic transducers capable of transmitting and receiving the ultrasonic waves, and configured to selectively activate the ultrasonic transducers through an electrical switch or the like. Generally, the ultrasonic prove can be categorized as either an endocavity type for insertion into a body cavity, or an external type placed against a body surface.

In view of the physical burden on patients, the endocavity ultrasonic probe should have a small (both in diameter and length) rigid tip section that encloses the ultrasonic transducers and a small diameter cable to be connected to the rigid tip section. The size of the rigid tip section is determined by the size and quantity of the ultrasonic transducers, while the external diameter of the cable is determined by the width and quantity of wires connected to the ultrasonic transducers. Therefore, if one or a few ultrasonic transducers were to be arranged, the rigid tip section would have the external diameter of as small as 3 to 6 mm.

When the quantity of the ultrasonic transducers is reduced, however, the resultant ultrasonic images will be of low resolution. In addition, if the ultrasonic transducers are arranged on a plane surface, the resultant ultrasonic images will have a narrow view angle. One effective countermeasure to these problems is the endocavity ultrasonic probe of radial type or convex type, in which a plurality of the ultrasonic transducers are arranged on a round surface to improve the resolution and the view angle of the ultrasonic images (see, for example, U.S. Patent No. 5,810,009). A radial type ultrasonic probe may have, for example, about 200 strip shaped (i.e. thin rectangular shape) ultrasonic transducers arranged on a cylindrical surface with about 9 mm outer diameter. Also, a convex type ultrasonic probe may have about 100 strip shaped ultrasonic transducers arranged on a convex surface with a curvature radius of 6 mm and a center angle of 120 degrees.

The dimension of the strip shaped ultrasonic transducer is decided, based chiefly on electrical impedance matching of the ultrasonic transducer to a drive circuit and the cable, such that transmitting sound pressure (or intensity of the ultrasonic wave in transmission) and receiver sensitivity can be enhanced as much as possible. Although it may depend partly on a dielectric constant and an electromechanical coupling coefficient of the piezoelectric material, the area of an ultrasonic wave transmitting/receiving surface of the ultrasonic transducer is generally in the range of 0.5 to 0.8 mm². In most cases, the dimension of the ultrasonic transducer is set to approximately 100 µm width by 5 mm length, in order to facilitate the arrangement of 100 to 200 ultrasonic transducer strips on the round surface. Therefore, the above radial type and convex type ultrasonic probes actually have a large rigid tip section with the external diameter of 10 to 12 mm. Reducing the dimension of the transmitting/receiving surface of each ultrasonic transducer will help reducing the size of the rigid tip section, but it degrades the transmit/receive performance (transmitting sound pressure and receiver sensitivity) at the same time.

Generally, such strip shaped ultrasonic transducer as described above requires high drive voltage of no less than 100V (usually 130V). Therefore, the adjacent ultrasonic transducers and adjoining wires in the cable must be definitely insulated from each other. It is also necessary to reduce noises and cross-talks generated due to a floating capacitance between the adjacent ultrasonic transducers or wires. For that reasons, the ultrasonic transducers have to be well spaced, and each wire becomes to have a thick insulating layer, causing to expand the external diameter of the rigid tip section.

### SUMMARY OF THE INVENTION

In view of the foregoing, an object of the present invention is to provide an endocavity ultrasonic probe which is made reduced in the thickness without lowering the performance of transmitting and receiving the ultrasonic waves.

To achieve the above object and other objects, an endocavity ultrasonic probe made according to the present invention is provided with ultrasonic transducers each of which has an alternate multilayer of electrode layers and at least two piezoelectric layers. The ultrasonic transducers are arranged on an external surface of a cylindrical or convex backing member, and radiate ultrasound waves toward an internal body part and then receive the echoes thereof. It is preferable to form the electrode layers to completely cover the upper and lower surfaces of the piezoelectric layer.

It is also preferable to provide a pair of side electrodes on the side faces of both the piezoelectric layers and electrode layers. The electrode layers alternately make electrical connection to a different one of the side electrodes. Favorably, insulators are provided between one of the side faces of each electrode layer and one of the side electrodes.

Each piezoelectric layer should be uniformly polarized in one direction across the whole region of the layer and, in this case, one of the two vertically adjacent piezoelectric layers across the electrode layer is polarized in an upward direction while the other is polarized in a downward direction.

According to the present invention, the electrode layers and at least two of the piezoelectric layers are alternately overlaid on each other. Thereby, the electrical impedance of the ultrasonic transducer is reduced, and thus adequate performance in transmitting and receiving the ultrasonic waves can be derived even if the ultrasonic transducer is minimized. This enables to reduce the thickness of the ultrasonic probe. In addition, since the ultrasonic transducers are able to drive with low drive voltage in this configuration, they can be spaced out at little intervals and the insulating layers of the wires can be made thin. These results also enable to reduce the thickness of the ultrasonic probe.

Since each of the electrode layer stretches across the entire upper or lower surface of the piezoelectric layer, the voltage applied in a polling process is uniformly distributed on the piezoelectric layer. Therefore, each piezoelectric layer is uniformly polarized in one direction, and therefore the performance of transmitting and receiving the ultrasonic waves will be improved.

### BRIEF DESCRIPTION OF THE DRAWINGS

For more complete understanding of the present invention, and the advantage thereof, reference is now made to the following descriptions taken in conjunction with the accompanying drawings, in which:
FIG.1 is a partial cutaway perspective view of a radial type endocavity ultrasonic probe;
FIG.2 is a perspective view of an ultrasonic transducer;
FIG.3 is an explanatory view of polarized piezoelectric layers in a polling process;
FIG.4 is a diagram of an electrical circuit connected to the ultrasonic transducers; and
FIG.5 is a cross sectional view of a convex type endocavity ultrasonic probe.

### DESCRIPTION OF THE PREFFERED EMBODIMENTS

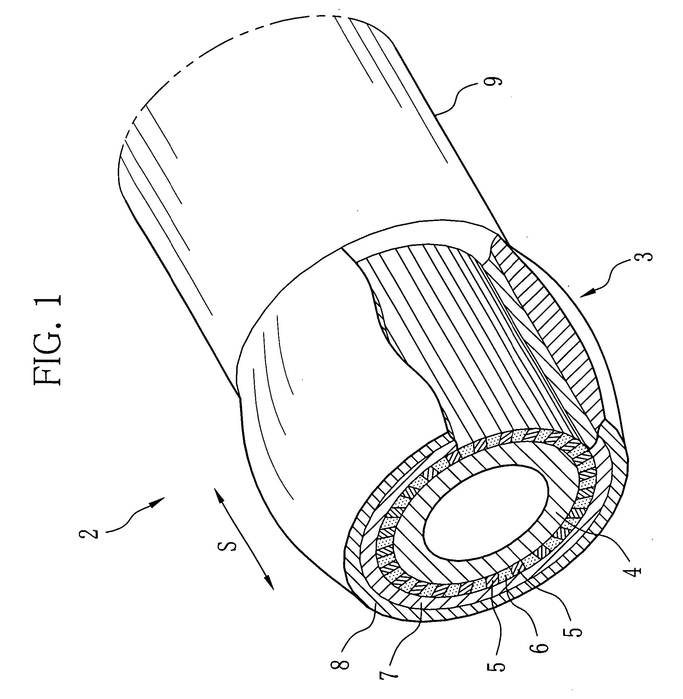

Referring to FIG.1, a radial type endocavity ultrasonic probe (hereinafter referred to as "ultrasonic probe") 2 is provided at a rigid tip section 3 with a cylindrical backing member (or supporting member) 4 made of ferrite rubber or the like, a plurality of strip shaped ultrasonic transducers 5 radially disposed on the external surface of the backing member 4, and filler 6 in between the adjacent ultrasonic transducers 5.

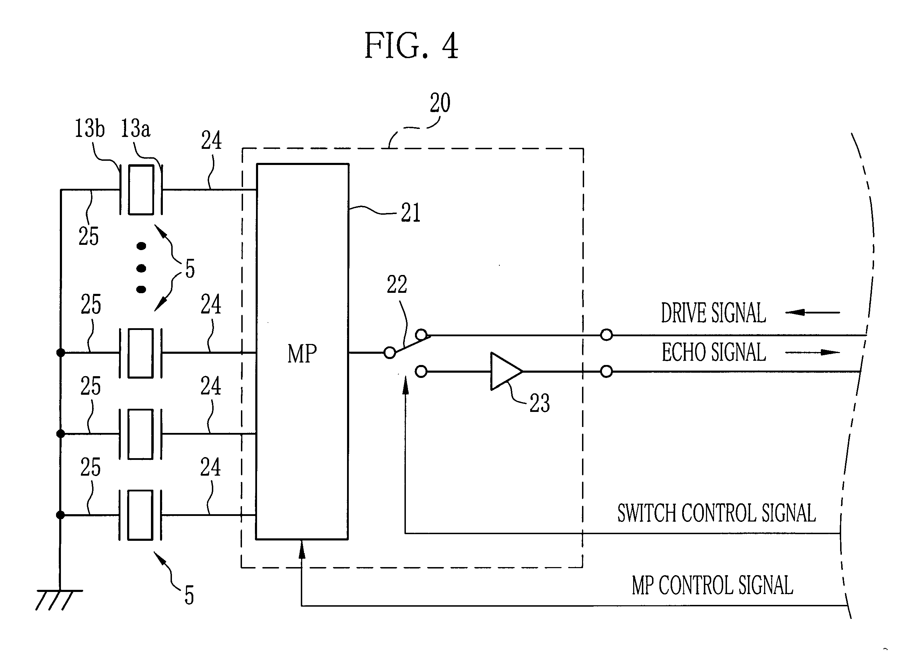

Provided on the outer side of the ultrasonic transducers 5 and the filler 6 is an acoustic matching layer 7, which is in turn covered with an acoustic lens 8. The acoustic matching layer 7 is a resin layer with a thickness equal to substantially one quarter wavelength of the ultrasonic waves radiated from the ultrasonic transducers 5, and compensates the difference in acoustic impedance between the ultrasonic transducers 5 and the body to be imaged. The acoustic lens 8 is also a resin layer swelling out in the middle with respect to a slice direction (i.e. "S" direction in the drawing), and focuses the ultrasonic waves from the ultrasonic transducers 5 on a focal point in the S direction. The rigid tip section 3 is attached to a cable 9, which contains wires 24 (see, FIG.4) connected with the ultrasonic transducers 5.

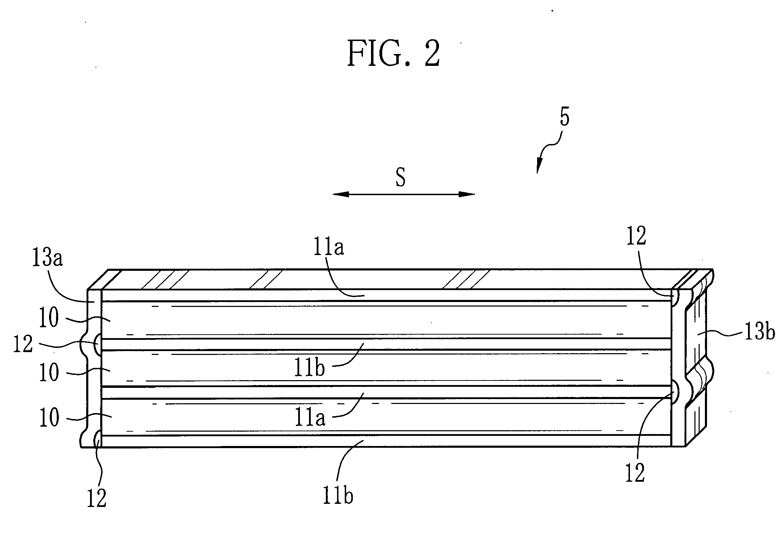

As shown in FIG.2, each of the ultrasonic transducers 5 is composed of three piezoelectric layers 10, electrode layers 11a and 11b formed respectively above and below the piezoelectric layer 10, insulating films 12 formed at one end of the electrode layers 11a and 11b, side electrodes 13a and 13b formed on the extreme ends on the slice direction. The electrode layer 11a is electrically connected with the side electrode 13a, but isolated from the side electrode 13b by the insulating film 12. Similarly, the electrode layer 11 b is electrically connected with the side electrode 13b, but isolated from the side electrode 13a by the insulating film 12.

The piezoelectric layers 10 are made of any suitable piezoelectric material, such as a Lead Zirconate Titanate (PZT), with approximately 85 µm thickness. The electrode layers 11a and 11b are made of a conductive material, such as platinum (Pt), with approximately 3 µm thickness. The insulating film 12 is made of an insulator, such as glass, with approximately 10 µm thickness. The side electrodes 13a and 13b is made of a conductive material, such as copper (Cu).

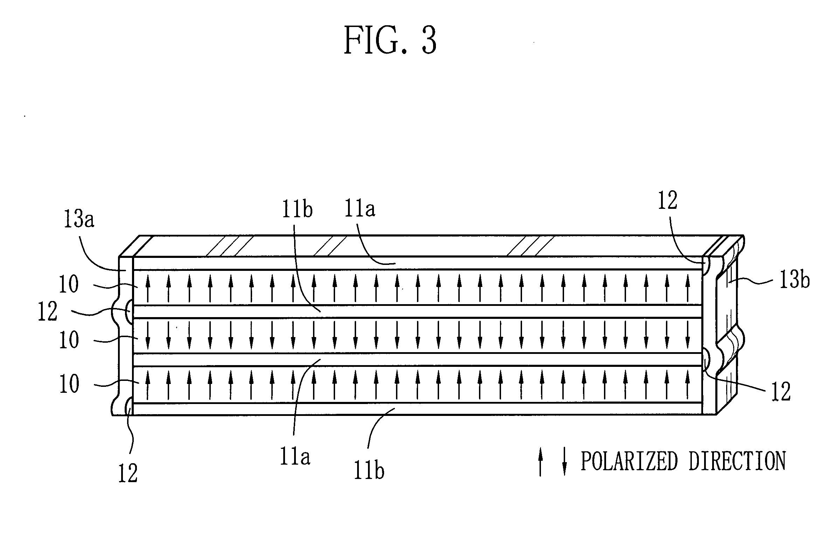

The electrode layers 11a and 11b are stretching across the entire upper and lower surfaces of the piezoelectric layer 10. This configuration of the electrode layers fosters the polarization of the piezoelectric layers in a polling process during the manufacture of the ultrasonic transducer 5. The polling process is for polarization reversal of the piezoelectric layer 10, where a voltage as high as 600V is applied between the electrode layers 11a and 11b. On account of the formation of the electrode layers 11a and 11b stretching across its entire upper and lower surfaces, the piezoelectric layer 10 will be evenly charged with the high voltage. Therefore, as shown in FIG. 3, each piezoelectric layer becomes polarized in one direction (indicated by arrows in the drawing), and the piezoelectric performance of the piezoelectric layer 10 is improved. In addition, the piezoelectric action will be effective throughout the piezoelectric layer 10, and the performance of the ultrasonic transducer 5 (radiation sound pressure and receiver sensitivity) is therefore improved.

As shown in FIG.4, an electric circuit 20 is included in a drive circuit (not shown), which is connected to the base end of the cable 9. The electric circuit 20 is provided with a multiplexer (MP) 21, a switch 22, and an amplifier 23. The multiplexer 21 follows MP control signals to selectively activate the ultrasonic transducers 5. The switch 22 follows switch control signals to switch between drive signals for the ultrasonic transducers 5 and echo signals from the ultrasonic transducers 5. The amplifier 23 amplifies the echo signals from the ultrasonic transducers 5. Note that the MP control signals and the switch, control signals are both output from an ultrasonic observation unit (not shown), to which the ultrasonic probe 2 is connected.

The side electrode 13a of each ultrasonic transducer 5 is connected through a wire 24 to the multiplexer 21. The side electrode 13b of each ultrasonic transducer 5 is grounded through a ground wire 25. The wires 24 run inside the cable 9. Alternatively, the side electrodes 13a may be connected to the ground wires 25 and the side electrodes 13b may be connected to the wires 24. In this case, the piezoelectric layers 10 are polarized in the opposite directions to those shown in FIG.3.

Next, the operation of the above configuration is explained. To obtain the ultrasonic image of an internal body part, the ultrasonic probe 2 is firstly inserted into a body cavity. When the rigid tip section 3 of the ultrasonic probe 2 arrives at an intended site inside the body and the imaging operation is initiated, the drive signals for the ultrasonic transducers 5 go through the switch 22 to the multiplexer 21. The multiplexer 21 selectively activates the ultrasonic transducers 5 to radiate ultrasonic waves with predetermined frequency (5 to 30 MHz, for example) toward the body part. The ultrasonic transducers 5 also receives echo waves from the body part. The received echo waves (or echo signals) are transmitted through the switch 22 to the amplifier 23. As selectively switching the ultrasonic transducers 5, the multiplexer 21 repeats this transmitting and receiving operation to scan the ultrasonic waves over the body part. The resultant echo signals are converted by the ultrasonic observation unit into ultrasonic images, which are displayed on a monitor screen or the like.

The ultrasonic probe 2 of the present invention is provided with the ultrasonic transducers 5 each of which has a multilayer structure of the three piezoelectric layers 10. With this multilayer structure, the ultrasonic transducer 5 becomes to have larger capacitance than a conventional transducer with a single piezoelectric layer 10. The difference in electrical impedance with the cable 9 or the drive circuit is thus reduced and the ultrasonic transducer 5 will show improved receiver sensitivity, which allows the drive voltage (i.e. voltage of the drive signal) to be lowered.

Such lowered drive voltage leads to reduce noises and cross talks resulting from the electric fields or floating capacitances between the adjacent ultrasonic transducers 5. It is therefore possible to arrange the ultrasonic transducers 5 at even smaller intervals on the surface of the backing member 4. When as many ultrasonic transducers 5 as conventionally required are used, the external diameter of the backing member 4 and then the dimension of the rigid tip section 3 become reduced. Also, it is possible to reduce the intervals between the wires 24 and to make thin the insulating layers thereon. The cable 9 is therefore can be thinned. Additionally, the length of the rigid tip section 3 can be reduced in the slice direction because the ultrasonic transducer 5 gives an adequate performance in transmitting and receiving the ultrasonic waves even if it is minimized. In this case, it is possible to reduce the thickness in the center of the optical lens 8 if the optical lens 8 has a conventional curvature. Furthermore, there will be no need of a large capacity power supply, and a cost reduction will be expected.

One exemplary embodiment of the present invention is now described to explain the difference in ultrasonic characteristics from conventional ultrasonic probes.

Firstly, as the ultrasonic probe 2 of the present invention, the backing member 4 with the length of 3.5 mm in the slice direction and the external diameter of 7.3 mm is provided, and 192 blocks of the ultrasonic transducers 5 are arranged, at 120 µm pitch, on the external surface of the backing member 4. Each ultrasonic transducer 5 has 90 µm width in the arranging direction, 270 µm thickness, and 3.5 mm length in the slice direction. The acoustic matching layer 7 has 70 µm thickness, and the acoustic lens 8 has 460 µm thickness at the central part in the slice direction (i.e. the maximum thickness). The ultrasonic probe 2 has the rigid tip section 3 whose length is 3.5 mm in the slice direction and whose central part has the external diameter of 8.9 mm in the slice direction.

In contrast, a conventional ultrasonic probe has the ultrasonic transducers each of which is composed of a single piezoelectric layer and the electrode layers on the upper and lower surfaces thereof. The backing member has 5.0 mm length in the slice direction and 9.0 mm external diameter. On the external surface of the backing member, 192 blocks of the ultrasonic transducers are arranged at 147 µm pitch. Each ultrasonic transducer has 110 µm width in the arranging direction, 270 µm thickness, and 5.0 mm length in the slice direction. The acoustic matching layer has 70 µm thickness, and the acoustic lens has 660 µm thickness at the central part in the slice direction (i.e. the maximum thickness). The ultrasonic probe of the conventional ultrasonic probe has the rigid tip section whose length is 5.0 mm in the slice direction and whose central part has the external diameter of 11.0 mm in the slice direction.

Then, both the ultrasonic probes 2 of the present invention and the conventional ultrasonic probe are disposed under water, approximately 10 cm away from a metal plate (not shown). In this state, pulses of drive voltage V1 are applied to each ultrasonic probe for radiation of the ultrasonic waves toward the metal plate, and the echo waves therefrom are received with the ultrasonic probe to evaluate its receiver sensitivity to the drive voltage V1.The receiver sensitivity (dB) is derived from the formula "20 x log(V2/V1)", in which V2 is the voltage, generated by the echo waves from the metal plate, between the electrodes of the ultrasonic transducer.

When applied with the drive voltage V1 of 130V and set to radiate the ultrasonic waves of 7.2 MHz, the conventional ultrasonic probe showed the receiver sensitivity of -57.5 dB. On the other hand, the ultrasonic probe 2 showed the drive voltage V1 of approximately 76V and the receiver sensitivity of -56.3 dB when it is set to radiate the 7.2 MHz ultrasonic waves and applied with as much drive voltage V1 as it can give the approximately equal receiver sensitivity to the conventional ultrasonic probe. It is recognized, from this result, that the present embodiment requires the much lesser drive voltage V1 to give the receiver sensitivity approximately equal to the conventional ultrasonic probe. Conversely speaking, the present embodiment gives the better receiver sensitivity than the conventional ultrasonic probe on the same drive voltage V1.

The above embodiment of the present invention uses the ultrasonic transducer constituted of the stack of three piezoelectric layers 10. However, the present invention is not limited to this constitution, and any suitable number (but not fewer than two) of the piezoelectric layers 10 may be stacked.

Although the side electrodes 13a and 13b of the above embodiment are placed on the opposite side faces in the slice direction, the side electrodes 13a and 13b can be placed on any two different side faces.

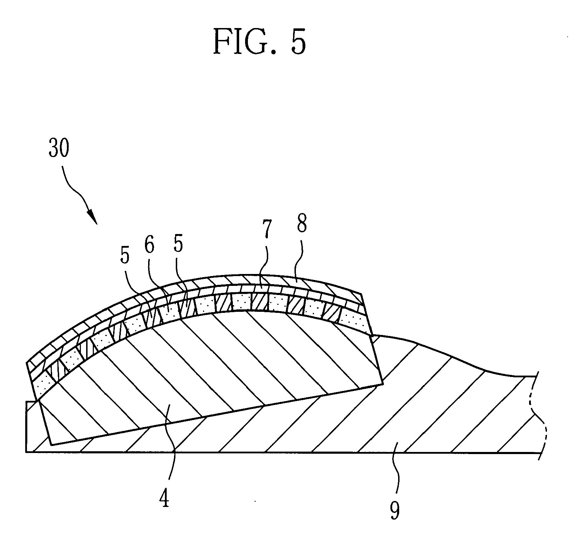

In the above embodiment, the ultrasonic probe 2 is of radial type in which the ultrasonic transducers 5 are radially arranged on the external surface of the cylindrical backing member 4. However, the present invention is not limited to this type and may be applied to the ultrasonic probes of convex type. FIG.5 shows a convex type ultrasonic probe 30 in which the ultrasonic transducers 5 are arranged on the external surface of the convex backing member 4.

Although the upper and lower surfaces of the piezoelectric layer 10 are completely covered with the electrode layers 11a and 11b in the above embodiment, it would not matter if the upper and lower surfaces are partly exposed because of unexpected pores or cracks on the electrode layers 11a and 11b, as long as the polarization in the polling process is not affected adversely.

As described so far, the present invention is not to be limited to the above embodiments, and all matter contained herein is illustrative and does not limit the scope of the present invention. Thus, obvious modifications may be made within the spirit and scope of the appended claims.

## Claims

1. An endocavity ultrasonic probe (2) inserted into a body cavity for radiating ultrasonic waves toward an internal body part and receiving echo waves, **characterized in** comprising:
a supporting member (4) formed either in a cylindrical or convex shape; and
a plurality of ultrasonic transducers (5) arranged on an external surface of said supporting member, each of said ultrasonic transducers including an alternate multilayer of electrode layers (11a, 11b) and at least two piezoelectric layers (10).

2. An endocavity ultrasonic probe as claimed in claim 1, wherein upper and lower surfaces of each of said piezoelectric layers (10) are completely covered with said electrode layers (11a, 11b).

3. An endocavity ultrasonic probe as claimed in claim 2 further comprising:
a pair of side electrodes (13a, 13b) provided on side faces of both said electrode layers (11a, 11b) and said piezoelectric layers (10), said electrode layers alternately making electrical connection to a different one of said side electrodes.

4. An endocavity ultrasonic probe as claimed in claim 3 further comprising:
insulators (12) formed between one of said side faces of said electrode layers (11a, 11b) and one of said side electrodes (10), said electrode layers alternately making contact with said insulators on opposite sides.

5. An endocavity ultrasonic probe as claimed in claim 4, wherein each of said piezoelectric layers (10) is uniformly polarized in one direction, one of the two of said piezoelectric layers disposed across said electrode layer being polarized in an upward direction while the other is polarized in a downward direction.
